# DEMANDE DE BREVET EUROPEEN

(11) **EP 4 684 793 A1**
(43) Date de publication de la demande: **28.01.2026**
(21) Numéro de dépôt: 25191541.9
(22) Date de dépôt: 24.07.2025
(51) Int. Cl.: A61K 36/481, A61P 37/04

(54) **PRÉPARATION D'UNE PLANTE DU GENRE ASTRAGALUS**

(30) Priorité: 25.07.2024 FR 2408260
(71) Demandeur: Larena, 49270 Oree D'Anjou (FR)
(72) Inventeur: DUBOURDEAUX, Michel, 03140 TAXAT (FR); GUINOBERT, Isabelle, 75011 Paris (FR); BARDOT, Valérie, 03140 TARGET (FR)
(74) Mandataire: Jacobacci Coralis Harle

(57) **Abrégé**

La présente invention concerne un préparation d'une plante du genre *Astragalus,* pour son utilisation comme immunorégulateur et/ou immunomodulateur chez des sujets humains ou animaux.

## Description

### Domaine technique de l'invention

La présente invention concerne le domaine des préparations de plantes, et plus particulièrement leur utilisation comme agents immunorégulateurs et/ou immunomodulateurs chez les sujets humains et animaux.

### Etat de la technique

Plusieurs approches ont été explorées pour assurer une régulation immunitaire, allant des médicaments synthétiques aux traitements à base de produits naturels.

Les immunomodulateurs et immunorégulateurs synthétiques, bien que largement utilisés, peuvent présenter des effets secondaires indésirables.

Il existe ainsi un intérêt croissant pour les thérapies alternatives, notamment celles basées sur des préparations de plantes.

Certaines préparations de plantes sont en effet connues pour contenir des composés bioactifs capables de moduler le système immunitaire. Ces composés peuvent agir à différents niveaux de la réponse immunitaire, y compris la stimulation des cellules immunitaires et la modulation de la réponse inflammatoire.

Une attention particulière est portée aux sujets âgés, chez qui le système immunitaire peut présenter une dysfonction liée à l'âge, augmentant ainsi leur susceptibilité aux infections, aux maladies auto-immunes immunes et au développement de pathologies liées à l'âge (syndrome métabolique, sarcopénie, démence, athérosclérose, cancer et ostéoporose). Les préparations de plantes offrent un potentiel important pour améliorer la réponse immunitaire chez les personnes âgées, contribuant ainsi à améliorer leur qualité de vie et à réduire les symptômes des maladies associées au vieillissement.

Cependant, malgré les progrès réalisés dans ce domaine, plusieurs défis persistent. La variabilité des profils phytochimiques des préparations de plantes peut influencer leur activité biologique, ce qui rend difficile la standardisation des formulations et la garantie de leur efficacité.

De plus, la compréhension des mécanismes d'action précis de ces préparations reste souvent incomplète, limitant ainsi leur application clinique.

Il est par conséquent toujours intéressant de proposer de nouvelles préparations de plantes spécifiques en tant qu'immunorégulateurs et immunomodulateurs.

### Présentation de l'invention

Afin de remédier à l'inconvénient précité de l'état de la technique, la présente invention propose une préparation d'une plante du genre *Astragalus,* de préférence sous forme d'un préparation aqueuse, pour son utilisation comme immunorégulateur et/ou immunomodulateur chez des sujets humains ou animaux.

Une telle préparation selon l'invention présente divers avantages, notamment :
- un effet immunorégulateur / immunomodulateur chez des sujets humains ou animaux : l'un des principaux avantages de cette préparation selon l'invention est son effet immunorégulateur / immunomodulateur, c'est-à-dire sa capacité à moduler le système immunitaire chez les humains et les animaux. En agissant comme un régulateur de l'immunité, cette préparation permet de maintenir un équilibre optimal du système immunitaire, par exemple, en atténuant le statut inflammatoire à l'état basal, en renforçant ainsi la capacité de l'organisme à répondre aux infections tout en limitant les réponses immunitaires dysfonctionnelles associées à l'âge ou, par exemple, aux maladies auto-immunes et aux allergies ;
- un effet sur les capacités de réponse antioxydante ; outre son effet immunorégulateur / immunomodulateur, cette préparation présente également des effets bénéfiques sur les capacités de réponse antioxydante de l'organisme ; en renforçant cette réponse de l'organisme, cette préparation peut contribuer à protéger les cellules et les tissus contre les dommages causés par les radicaux libres, réduisant ainsi le risque de maladies chroniques liées au vieillissement et à l'inflammation.

D'autres caractéristiques non limitatives et avantageuses du produit conforme à l'invention, prises individuellement ou selon toutes les combinaisons techniquement possibles, sont les suivantes :
- ladite utilisation comme immunorégulateur et/ou immunomodulateur comprend : une utilisation comme immunorégulateur et/ou immunomodulateur du système immunitaire en situation basale, et/ou une utilisation comme immunorégulateur et/ou immunomodulateur du système immunitaire lors d'une réponse immunitaire anti-infectieuse, en particulier bactérienne et/ou virale et/ou une utilisation comme immunorégulateur et/ou immunomodulateur du système immunitaire lors d'une vaccination ;
- les sujets sont choisis chez les sujets âgés, avantageusement ayant atteint les deux tiers de leur durée de vie, par exemple les sujets humains âgés, par exemple les sujets humains ayant au moins 50 ans, de préférence au moins 60 ans, de préférence au moins 65 ans ;
- ladite plante du genre *Astragalus* est choisie parmi *Astragalus alopecuroides L., Astragalus alopecurus Pall., Astragalus alpinus L., Astragalus australis (L.) Lam., Astragalus austriacus Jacq., Astragalus baionensis Loisel., Astragalus boeticus L., Astragalus cicer L., Astragalus crenatus Schult., Astragalus frigidus L., Astragalus glycyphyllos L., Astragalus hamosus L., Astragalus hypoglottis L., Astragalus membranaceus var mongholicus* (Bunge) P.K. Hsiao , *Astragalus membranaceus* (Fisch.) Bunge, *Astragalus membranaceus* Moench,. *Astragalus monspessulanus L., Astragalus mongholicus* Bunge, *Astragalus tragacantha L., Astragalus onobrychis L., Astragalus sempervirens Lam ;*
- ladite préparation est issue d'une plante sèche ou fraîche ;
- ladite préparation est issue de la racine de ladite plante ;
- ladite préparation est issue d'un procédé comprenant les étapes suivantes :
   -- une étape de broyage de la plante, dont les particules présentent avantageusement des dimensions allant de 5 mm à 10 mm,
   -- au moins une étape d'extraction dans un milieu aqueux, pendant au moins 1h, par exemple allant de 1h à 10h, à une température allant de 50 à 90°C,
   -- une étape de filtration pour récupérer une préparation aqueuse intermédiaire,
   -- une étape de concentration (par exemple évaporateur rotatif) et de séchage (par exemple lyophilisation ou atomisation), pour obtenir ladite préparation aqueuse.

La présente invention concerne encore une composition pharmaceutique ou non-pharmaceutique, contenant une préparation aqueuse selon l'invention, pour son utilisation comme immunorégulateur et/ou immunomodulateur chez des sujets humains ou animaux.

De préférence :
- la composition contient en outre l'un au moins des ingrédients suivants : au moins une préparation d'algue, au moins une préparation de plante, des probiotiques, des postbiotiques, des vitamines, des minéraux ou tout autre ingrédient nutritionnel ;
- la composition est adaptée à une utilisation dans l'industrie cosmétologique, agro-alimentaire, de la nutrition, des compléments alimentaires, de la nutrition animale, dans une utilisation non-thérapeutique.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée.

La présente invention concerne ainsi, de manière générale, les préparations d'une plante du genre *Astragalus* (dites encore « préparations d'astragale »), de préférence sous forme d'une préparation aqueuse, pour leurs utilisations comme immunorégulateur et/ou immunomodulateur chez des sujets humains ou animaux.

### Astragalus

Le genre *Astragalus* englobe des plantes de la famille Fabacées.

Ce genre *Astragalus* englobe en particulier des plantes couramment appelées « astragale », « fausse réglisse » ou « Huang Qi ».

Encore de manière générale, les plantes du genre *Astragalus* sont encore définies sur le site Internet World Flora Online (WFO) Plant List, https://wfoplantlist.org/.

Selon une forme de réalisation préféré, la plante du genre *Astragalus* est choisie parmi *Astragalus alopecuroides L., Astragalus alopecurus Pall., Astragalus alpinus L., Astragalus australis (L.) Lam., Astragalus austriacus Jacq., Astragalus baionensis Loisel., Astragalus boeticus L., Astragalus cicer L., Astragalus crenatus Schult., Astragalus frigidus L., Astragalus glycyphyllos L., Astragalus hamosus L., Astragalus hypoglottis L., Astragalus membranaceus var mongholicus (Bunge) P.K. Hsiao , Astragalus membranaceus (Fisch.) Bunge, Astragalus membranaceus Moench,. Astragalus monspessulanus L., Astragalus mongholicus Bunge, Astragalus tragacantha L., Astragalus onobrychis L., Astragalus sempervirens Lam.*

### Préparation d'une plante du genre Astragalus et son procédé d'extraction

De préférence, la préparation selon l'invention est issue d'une plante sèche ou d'une plante fraîche.

De manière générale, cette préparation est encore avantageusement issue de la racine de cette plante.

Par « préparation de plante », on englobe avantageusement les préparations, sous forme sèche ou liquide, obtenues à partir des matières premières végétales, notamment en les réduisant en poudre ou en les traitant par un procédé d'extraction, de distillation, d'expression, de fractionnement, de purification, de concentration ou de fermentation.

Par « préparation », on entend également les extraits, sous forme sèche ou liquide.

De préférence, la préparation selon l'invention, sous une forme aqueuse, est issue d'un procédé comprenant avantageusement les étapes suivantes :
- une étape de broyage de la plante, par exemple de sa racine,
- au moins une étape d'extraction dans un milieu aqueux, pendant au moins 1h,
- une étape de filtration pour récupérer une préparation aqueuse intermédiaire,
- une étape de concentration et de séchage, pour obtenir ladite préparation aqueuse selon l'invention.

L'étape de broyage est mise en œuvre de sorte à obtenir des particules qui présentent avantageusement des dimensions allant de 5 mm à 10 mm (avantageusement combiné avec une opération de calibration).

Cette dimension des particules est avantageusement déterminée par tamisage, selon le procédé décrit dans Eco-extraction du végétal, Procédés innovants et solvants alternatifs, Farid Chemat - Collection Technique et ingénierie - EEA, Éditeur Dunod, Parution 06/11/2015.

L'étape d'extraction est par exemple mise en œuvre sur une durée allant de 1h à 10h, à une température allant de 50 à 90°C.

Cette étape d'extraction consiste avantageusement en une étape de digestion des particules dans un solvant aqueux.

La digestion consiste avantageusement à maintenir en contact les particules avec de l'eau, à une température inférieure à celle de l'ébullition, mais supérieure à la température ambiante.

Par exemple, l'homme du métier peut se référer au document suivant :
- Plantes Therapeutiques. Tradition, Pratique Officinale, Science Et Therapeutique, Correspond A La 3eme Edition Allemande, Date de parution 26/05/1999 Editeur Tec & Doc (Editions) ISBN 2-7430-0267-0,
- Arrêté du 5 juin 2000 portant additif n° 46 à la Pharmacopée, NOR : MESM0021767A, Journal officiel du 19 juillet 2000.

Par ailleurs, l'étape de concentration est par exemple mise en œuvre au moyen d'un évaporateur sous vide.

L'étape de séchage consiste par exemple en un procédé de lyophilisation ou d'atomisation, pour obtenir la préparation aqueuse selon l'invention sous une forme sèche.

L'homme du métier peut encore se référer aux documents suivants pour le paramétrage de ces étapes successives :
- Plantes Therapeutiques. Tradition, Pratique Officinale, Science Et Therapeutique, Correspond A La 3eme Edition Allemande, Date de parution 26/05/1999 Editeur Tec & Doc (Editions) ISBN 2-7430-0267-0,
- Arrêté du 5 juin 2000 portant additif n° 46 à la Pharmacopée, NOR : MESM0021767A, Journal officiel du 19 juillet 2000.

De manière générale, la préparation aqueuse selon l'invention contient :
- des polysaccharides,
- des saponines, par exemples des astragalosides, et
- des flavonoïdes, par exemple la formononétine.

Par « polysaccharides », on entend avantageusement un polysaccharide de la famille des glucanes dont la teneur est comprise entre 1 et 20 %.

Par exemple, les polysaccharide de la famille des glucanes englobent majoritairement une chaîne de glucoses liés en (1,4).

En d'autres termes, les polysaccharide de la famille des glucanes englobent en particulier les α-D-glucanes ramifiés, avec une chaîne principale en (1,4), occasionnellement interrompue par des liaisons (1,3) et branchée via des liaisons (1,6).

Par « astragolosides », on entend avantageusement des glycosides cycloartanes.

Ces composés sont par exemple décrits dans le document Molecules, 2014, 19. 18850-18880, Xiaoxia Li *et al.*

De préférence, on englobe les astragolosides I à VII, et en particulier l'astragoloside IV.

La formule chimique structurelle de l'astragoloside IV est la suivante :

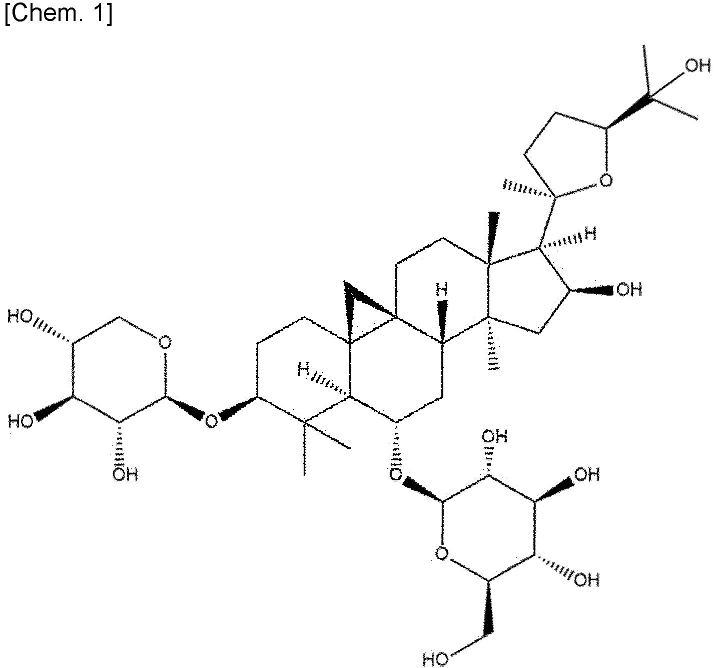

Les astragolosides englobent encore avantageusement les cycloastragénols.

Par « formononétine », on entend un flavonoïde de type isoflavone.

Ces composés sont par exemple décrits dans le document suivant Molecules, 2014, 19. 18850-18880, Xiaoxia Li *et al.*

### Utilisation comme immunorégulateur et/ou immunomodulateur

La présente invention concerne donc la préparation selon l'invention pour son utilisation comme immunorégulateur et/ou immunomodulateur.

Par « immunorégulateur », on entend en particulier une préparation qui agit pour réguler l'activité du système immunitaire. Il peut contribuer à maintenir un équilibre approprié entre les différentes composantes de la réponse immunitaire en supprimant les réponses immunitaires excessives ou inappropriées, ou en stimulant les réponses immunitaires affaiblies.

Par « immunomodulateur », on entend un agent capable de moduler l'activité du système immunitaire en ajustant sa réponse en fonction des besoins spécifiques de l'organisme. Cela englobe l'amplification ou l'atténuation sélective de certaines composantes de la réponse immunitaire, en fonction du contexte pathologique ou physiologique.

De préférence encore, la présente invention comprend encore la préparation selon l'invention pour une utilisation comme immunorégulateur et/ou immunomodulateur du système immunitaire en situation basale.

Par « situation basale », on entend avantageusement une situation sans infection. Cela signifie un état dans lequel il n'y a pas de présence détectable de micro-organismes pathogènes ou d'infections actives dans l'organisme ou l'environnement de l'individu.

En d'autres termes encore, la situation basale, sans infection, est un état où l'individu ne présente aucun signe d'infection, c'est-à-dire l'absence de pathogènes infectieux détectables.

En effet, chez les sujets âgés, il est courant d'observer une inflammation chronique de bas niveau, même en l'absence d'infections actives. Cette inflammation latente peut être causée par divers facteurs liés au vieillissement et peut contribuer à des symptômes inflammatoires chroniques qui affectent la qualité de vie.

L'application de la préparation aqueuse dans cette population peut avoir des effets bénéfiques significatifs. En atténuant le contexte inflammatoire latent, la préparation peut réduire les symptômes associés à l'inflammation chronique. Cela inclut la diminution de la douleur, de la fatigue, et d'autres symptômes inflammatoires qui peuvent altérer la qualité de vie.

A cette fin, la préparation est apte à interagir avec les différentes composantes du système immunitaire et réguler leur activité de manière à maintenir un équilibre approprié.

Il agit avantageusement sur la réponse immunitaire, en stimulant ou en supprimant sélectivement certaines réponses immunitaires selon les besoins de l'organisme.

Par exemple, il peut aider à renforcer la réponse immunitaire en cas d'immunosuppression légère ou à atténuer une réponse immunitaire excessive dans des conditions d'hyperactivité immunitaire, telles que les allergies ou les maladies auto-immunes.

En agissant de manière ciblée et équilibrée, la préparation selon l'invention contribue à maintenir la santé et le fonctionnement optimal du système immunitaire en situation basale, favorisant ainsi le bien-être général du sujet.

De manière alternative ou complémentaire, la présente invention comprend encore la préparation selon l'invention pour une utilisation comme immunorégulateur et/ou immunomodulateur du système immunitaire lors d'une réponse immunitaire anti-infectieuse, en particulier bactérienne et/ou virale.

En présence d'une infection, le système immunitaire est activé pour combattre l'agent pathogène, déclenchant une cascade complexe de réactions immunitaires.

La préparation selon l'invention intervient dans ce processus en modulant la réponse immunitaire de manière à renforcer l'efficacité de la défense de l'organisme tout en limitant les dommages causés par une réponse inflammatoire excessive.

En offrant un soutien immunomodulateur ciblé et efficace, la préparation selon l'invention peut aider à accélérer la résolution de l'infection, à réduire la gravité des symptômes et à prévenir les complications associées aux infections bactériennes et virales.

De manière alternative ou complémentaire, la présente invention comprend encore la préparation selon l'invention pour une utilisation comme immunorégulateur et/ou immunomodulateur lors d'une vaccination (en combinaison avec l'administration d'un vaccin).

Cette application est particulièrement avantageuse car elle permet d'optimiser la réponse immunitaire chez un individu dans le cadre d'une vaccination, et de préférence la réponse IgG à la vaccination antigrippale chez ces sujets fragiles immunosénescents.

De manière surprenante, la préparation selon l'invention possède un effet adjuvant sur la réponse vaccinale. Un adjuvant est une substance qui, lorsqu'elle est administrée en même temps qu'un antigène, améliore la réponse immunitaire à cet antigène. Cet effet est particulièrement observé lors de l'administration par voie orale (*per os*) chez des sujets âgés vaccinés contre la grippe.

L'administration *per os* de la préparation d'astragale, en combinaison avec un vaccin, permet d'augmenter l'efficacité de ce dernier, en stimulant une réponse immunitaire plus robuste et durable. Cela est particulièrement bénéfique pour les populations âgées, dont le système immunitaire est souvent moins réactif aux vaccins traditionnels.

La préparation selon l'invention est avantageusement administrée avant et/ou après la vaccination, chacune sur une période allant de 7 à 15 jours. Cette période d'administration permet de maximiser l'effet immunorégulateur et immunomodulateur de la préparation, en assurant que le système immunitaire est optimalement préparé pour répondre au vaccin et pour maintenir cette réponse efficace. Cette activité englobe un renforcement et/ou une prolongation de la réponse immunitaire induite par le vaccin.

L'utilisation de cette préparation en tant qu'immunorégulateur et/ou immunomodulateur présente plusieurs avantages spécifiques lorsqu'elle est utilisée en combinaison avec des vaccins contre les maladies virales :
- efficacité accrue des vaccins viraux : L'effet adjuvant de l'extrait d'astragale peut améliorer la réponse immunitaire contre une gamme de virus, tels que le virus de la grippe ou le virus responsable de la COVID. Cela permet de renforcer la protection vaccinale et de réduire la morbidité et la mortalité associées à ces infections virales ;
- amélioration de la réponse immunitaire chez les populations vulnérables : Les personnes âgées, les immunodéprimés et d'autres groupes vulnérables ont souvent une réponse vaccinale diminuée. L'administration parallèle de la préparation peut aider à surmonter cette limitation et à fournir une protection plus efficace contre les maladies virales.
- administration facile et non invasive : L'administration *per os* est particulièrement avantageuse pour les programmes de vaccination, car elle est facile à administrer et bien tolérée par les patients.

La préparation selon l'invention peut ainsi constituer également un adjuvant, en combinaison avec des préparations vaccinales contre les maladies virales, offrant une méthode pour améliorer la réponse vaccinale et fournir une protection accrue contre les infections virales, en particulier chez les populations les plus vulnérables.

L'administration de la préparation sur une période avant et/ou après la vaccination assure une optimisation de la réponse immunitaire, renforçant ainsi l'efficacité globale du vaccin et permettant de prolonger la durée de la réponse immunitaire.

### Sujet

Par « sujets humains », on entend en particulier les sujets humains adultes.

Par « sujets animaux », on entend en particulier les animaux de compagnie tels que les chiens et les chats, ainsi que les animaux d'élevage tels que les bovins, les porcins, les ovins et les équidés.

De manière plus générale, les sujets sont de préférence choisis chez les sujets âgés, avantageusement ayant atteint les deux tiers de leur espérance de vie.

Par « âgé », on englobe encore les sujets ayant un âge supérieur à 66% de leur espérance de vie, de préférence encore 70%, 80%, 90%.

Par « espérance de vie » ou « espérance de vie à la naissance », on entend avantageusement la durée de vie moyenne (autrement dit l'âge moyen au décès) d'une génération fictive soumise aux conditions de mortalité de l'année. Elle caractérise la mortalité indépendamment de la structure par âge.

Les sujets âgés représentent en effet une population particulièrement vulnérable aux dysfonctionnements du système immunitaire liés à l'âge.

Les sujets âgés sont souvent confrontés à une immunité affaiblie due à un certain nombre de facteurs, notamment le vieillissement naturel du système immunitaire.

La préparation vise ainsi à renforcer et à soutenir leur système immunitaire affaibli, contribuant ainsi à améliorer leur résistance aux infections et à réduire le risque de complications associées.

L'utilisation de la préparation aqueuse chez les sujets âgés peut également aider à atténuer le contexte inflammatoire latent et les symptômes inflammatoires chroniques souvent observés dans cette population, améliorant ainsi leur qualité de vie globale.

De préférence, par « sujet », on entend ainsi les sujets humains âgés, par exemple les sujets humains ayant au moins 50 ans, de préférence au moins 60 ans, de préférence au moins 65 ans.

### Composition

La présente invention concerne encore la composition pharmaceutique ou la composition non-pharmaceutique, contenant une préparation aqueuse selon l'invention, pour son utilisation comme immunorégulateur et/ou immunomodulateur chez des sujets humains ou animaux.

La composition peut contenir en outre avantageusement l'un au moins des ingrédients suivants : au moins une préparation d'algue, au moins une préparation de plante, des probiotiques, des postbiotiques, des vitamines, des minéraux ou tout autre ingrédient nutritionnel.

La préparation d'algue peut être choisie parmi différentes espèces d'algues, telles que le porphyra, une algue rouge également connue sous le nom de nori, la spiruline (parfois désigné comme bactérie), la chlorelle ou une algue brune.

La préparation de plante peut provenir de différentes plantes, telles que le boswellia, le curcuma, les feuilles de cassis, la saule, la reine des prés, la prêle, l'harpagophytum, la scrofulaire, le cresson du Para (*Acmella oleracea*), le cyprès (*Cupressus sempervirens*), l'échinacée (*Echinacea spp*.).

De manière générale, par « souche probiotique », on englobe en particulier les microorganismes vivants qui, lorsqu'ils sont ingérés en quantité suffisante, exercent des effets positifs sur la santé, au-delà des effets nutritionnels traditionnels.

La souche probiotique convenant à l'invention est physiologiquement acceptable. En d'autres termes, cette souche probiotique peut être administrée sans risques à l'animal ou l'homme.

Les souches probiotiques peuvent notamment être des lactobacilles (bactéries du genre *Lactobacillus*), bifidobactéries (bactéries du genre *Bifidobacterium*), streptocoques (bactéries du genre *Streptococcus*) ou lactocoques (bactéries du genre *Lactococcus*).

Encore de manière générale, des exemples spécifiques de microorganismes probiotiques sont *Bifidobacterium adolescentis, Bifidobacterium animalis* (subsp. *animalis* ou *lactis), Bifidobacterium bifidum, Bifidobacterium breve, Bifidobacterium longum* (subsp. *longum* ou *infantis*), *Bifidobacterium pseudocatenulatum, Lactobacillus acidophilus, Lactobacillus amylovorus, Lacticaseibacillus casei, Lacticaseibacillus rhamnosus, Levilactobacillus brevis, Lactobacillus crispatus, Lactobacillus delbrueckii (subsp bulgaricus* ou *delbrueckii* ou *lactis), Limosilactobacillus fermentum, Lactobacillus helveticus, Lactobacillus gallinarum, Lactobacillus gasseri, Lactobacillus paragasseri, Lactobacillus johnsonii, Lacticaseibacillus paracasei, Lacticaseibacillus plantarum, Lacticaseibacillus reuteri, Ligilactobacillus salivarius, Companilactobacillus alimentarius, Latilactobacillus curvatus, Latilactobacillus sake, Enterococcus (faecalis, faecium), Lactococcus lactis (*subsp *lactis* ou *cremoris), Leuconstoc mesenteroides, Pediococcus acidilactici, Sporolactobacillus inulinus, Streptococcus salvarius* subsp. *thermophilus, Staphylococccus carnosus, Staphylococcus xylosus, Saccharomyces (cerevisiae* ou encore *boulardii), Bacillus (cereus var toyo ou subtilis), Bacillus coagulans, Bacillus licheniformis, Propionibacterium freudenreichii* et leurs mélanges.

Plus particulièrement, il s'agit de microorganismes probiotiques issus du groupe des bactéries lactiques, appartenant notamment aux genres *Lactobacillus* et/ou les *Bifidobacterium.* A titre illustratif de ces bactéries lactiques, on peut plus particulièrement citer les espèces *Lactobacillus johnsonii, Lactobacillus reuteri, Lactobacillus rhamnosus, Lactobacillus paracasei, Lactobacillus casei ou Bifidobacterium bifidum, Bifidobacterium breve, Bifidobacterium longum, Bifidobacterium animalis, Bifidobacterium lactis, Bifidobacterium infantis, Bifidobacterium adolescentis ou Bifidobacterium pseudocatenulatum* et leurs mélanges.

De préférence, la préparation est mélangée avec 4 souches appartenant aux espèces *Bifidobacterium longum, Lactobacillus helveticus, Lactococcus lactis* et *Streptococcus thermophilus.*

De manière générale, ladite au moins une souche probiotique est avantageusement choisie parmi les souches probiotiques vivantes.

Par forme « vivante », on entend une forme dotée de la capacité à se multiplier sous réserve de la placer dans un environnement propice à la récupération de cette capacité.

Ainsi, au sens de la présente invention, le terme vivant couvre l'état dit dormance dans lequel les microorganismes peuvent être placés suite à un traitement physicochimique tel que par exemple la lyophilisation.

De manière générale, la souche probiotique est avantageusement mise en œuvre sous une forme lyophilisée.

Ce type de formulation possède les avantages d'être facilement accessible, de mise en œuvre aisée et de ne soulever aucune difficulté et/ou contrainte en termes de stockage. Par ailleurs, il est compatible avec le conditionnement de microorganismes à l'état de dormance.

Cette lyophilisation peut être réalisée selon des méthodes conventionnelles ou selon une méthode décrite par exemple dans le document FR-3 103 827.

La concentration en souche probiotique, dans la composition, est par exemple de 10⁷ à 10¹² UFC par dose quotidienne.

La concentration en souche probiotique dans la composition peut être d'une manière générale une quantité efficace permettant d'obtenir l'effet recherché. Cette quantité peut être déterminée par la personne du métier au moyen de ses connaissances générales et par des tests usuels. Cette quantité peut par exemple être, pour une application topique, en dose journalière exprimée en CFU, d'environ 10⁶ à environ 10¹², de préférence au moins 10⁶ à environ 10¹⁰, de préférence d'environ 10⁸ à environ 10⁹. Elle peut être alternativement d'environ 10⁵ à environ 10¹² organismes/g de produit.

La souche probiotique peut encore être incluse à hauteur de de 0,01% à 50% dans la composition, par exemple entre 0,01% et 40%, ou entre 0,01% et 30%, ou entre 0,01% et 10%, ou entre 0,01% et 5%, ou entre 0,01% et 1%, par exemple environ 0,3% (poids/poids).

La composition peut également contenir des postbiotiques, dits encore « préparations de micro-organismes inanimés et/ou de leurs composants qui confère un bénéfice santé à son hôte », tel que définis dans le document Front Microbiol. 2024 ;14:1324565, Vinderola G et al. Ces micro-organismes peuvent être avantageusement choisis parmi les souches appartenant aux espèces précitées.

Le terme « inanimé » désigne des souches ayant subi un traitement visant à les tuer. De tels traitements peuvent consister, à titre d'exemple non limitatif, en un traitement en autoclave, par ultra-sons, une homogénéisation haute pression ou encore un choc osmotique.

Les composants d'un postbiotique incluent avantageusement des cellules microbiennes inanimées intactes ou des fragments de celles-ci ou des fragments de structures (composants des parois cellulaires ou des protéines membranaires, par exemple), avec ou sans métabolites synthétisés par des bactéries.

La composition peut encore contenir d'autres types d'ingrédients, par exemple des vitamines, des minéraux ou tout autre ingrédient nutritionnel, des prébiotiques, des métabiotiques, des beta-glucanes ou autres polysaccharides.

Plus particulièrement, la composition peut contenir des ingrédients nutraceutiques comme la vitamine C, la glucosamine, la chondroïtine, le palmitoyléthanolamide ou des acides gras.

La composition selon l'invention contient avantageusement une combinaison d'ingrédients adaptés à une utilisation dans l'industrie pharmaceutique, cosmétologique, agro-alimentaire, de la nutrition (y compris les denrées alimentaires destinées à des fins médicales spéciales - DADFMS), des compléments alimentaires, de la nutrition animale.

La composition selon l'invention comprend ainsi avantageusement :
- une préparation selon l'invention, pour son utilisation chez des sujets humains ou animaux, et
- au moins un support ou un excipient, acceptable en fonction du l'utilisation envisagée.

La préparation de la présente invention peut être préparée sous la forme d'une composition pharmaceutique, qui peut être une formulation telle que des comprimés, des capsules, des poudres, des granules, des solutions, des pastilles, des gelées, des préparations de crème, des sirops, des suspensions, des teintures, des aérosols et autres.

La préparation de la présente invention peut également être préparée sous la forme d'une composition non-pharmaceutique.

La composition non-pharmaceutique englobe les compositions cosmétologiques, les compositions agro-alimentaires, les compositions nutritionnelles, les compléments alimentaires, les compositions pour la nutrition animale.

La composition non-pharmaceutique peut être préparée par des techniques de préparation généralement connues, et des additifs acceptables peuvent être ajoutés à la composition non-pharmaceutique.

Par exemple, la composition non-pharmaceutique selon l'invention consiste avantageusement en un complément alimentaire.

Par « complément alimentaire », on entend avantageusement les compléments alimentaires qui sont soumis à l'ensemble des dispositions générales du droit alimentaire mais aussi aux règles spécifiques définies par la directive 2002/46/CE du Parlement européen et du Conseil du 10 juin 2002 relative au rapprochement des législations des États membres concernant les compléments alimentaires, transposée en droit français par le décret n°2006-352.

Par « compléments alimentaires », on englobe avantageusement les denrées alimentaires dont le but est de compléter le régime alimentaire normal et qui constituent une source concentrée de nutriments ou d'autres substances ayant un effet nutritionnel ou physiologique seuls ou combinés, commercialisés sous forme de doses, à savoir les formes de présentation telles que les ampoules de liquide, les flacons munis d'un compte-gouttes et les autres formes analogues de préparations liquides.

De manière générale, la composition selon l'invention est adaptée à une administration par voie orale, *per os* ou topique.

Encore de manière générale, la préparation selon l'invention est avantageusement destinée à des sujets qui présentent diverses pathologies inflammatoires chroniques, des symptômes liés à un système immunitaire affaibli, ou des conditions physiologiques associées au vieillissement, telles que l'immunosénescence. Elle peut également être intéressante pour des sujets recevant un vaccin.

De préférence, la préparation selon l'invention est administrée selon une posologie adaptée, ou des préconisations d'utilisation adaptées, à chaque cas spécifique et à chaque besoin individuel.

Cette posologie peut être :
- ponctuelle (ou à court terme ou sous la forme d'un traitement symptomatique), sur quelques jours, pour traiter (ou au moins limiter les symptômes dans) des épisodes aigus d'inflammation ou pour renforcer temporairement le système immunitaire en cas de stress ou d'exposition à des agents pathogènes, ou pour optimiser la réponse immunitaire dans le cadre d'une vaccination, ou
- chronique (ou à long terme ou sous la forme d'un traitement de fond), pour un usage continu visant à maintenir une régulation immunitaire optimale à long terme, notamment chez les sujets âgés ou chez ceux souffrant de pathologies inflammatoires chroniques.

### Utilisation non-thérapeutique

La présente invention englobe encore une utilisation non-thérapeutique d'une préparation selon l'invention, pour son utilisation comme immunorégulateur et/ou immunomodulateur chez des sujets humains ou animaux :
- de manière ponctuelle, pour prévenir/limiter des épisodes aigus d'inflammation, notamment en cas de stress ou d'exposition à des agents pathogènes, ou
- de manière chronique pour maintenir une régulation immunitaire/inflammatoire optimale à long terme, notamment chez les sujets âgés avec ou sans maladie liée à l'âge.

Bien entendu, diverses autres modifications peuvent être apportées à l'invention dans le cadre des revendications annexées.

### Exemples

### Préparation aqueuse d'astragale

La préparation est issue d'un procédé comprenant les étapes suivantes :
- une étape de broyage de la plante, dont les particules présentent avantageusement des dimensions allant de 5 mm à 10 mm, par exemple des racines d'*Astragalus mongholicus* Bunge,
- au moins une étape d'extraction dans un milieu aqueux, pendant 2h, à une température de 80°C,
- une étape de filtration pour récupérer une préparation aqueuse intermédiaire,
- une étape de concentration (évaporateur rotatif) et de séchage (atomisation), pour obtenir la préparation aqueuse sous forme d'une poudre.

### Exemple 1 : activité de la préparation aqueuse d'astragale sur le système immunitaire d'animaux âgés en situation basale

Des souris mâles C57BL/6 âgées de 18 mois ont reçu par gavage quotidien une dose de 40mg/kg de la préparation aqueuse d'astragale.

Au bout de 15 jours, le sang, le liquide péritonéal et les urines sont récupérés pour analyser plusieurs paramètres reflétant l'état du système immunitaire : détermination par cytométrie de flux des populations de cellules immunitaires (nombre et proportion), dosage par ELISA de médiateurs de la réponse inflammatoire, expression de gènes impliqués dans l'inflammation et le stress oxydant par RT-QPCR.

Un groupe de souris de même âge ne recevant pas la préparation, subissant les mêmes analyses, est employé comme comparateur pour évaluer l'impact de la préparation d'astragale.

Chez des souris âgées non infectées, la préparation aqueuse d'astragale a pour effet°:
- de moduler les populations immunitaires innées et adaptatives en faveur d'une réponse anti-infectieuse mieux préparée (augmentation des monocytes patrouilleurs, du ratio Th1/Th2, des macrophages SPM, baisse des macrophages LPM),
- de réduire le statut inflammatoire (baisse de la concentration sérique de SAP et d'IL-6, baisse de la concentration dans les urines de PGE2),
- de réduire l'expression de médiateurs (IL-1b, IL-6, CCl2) et d'enzymes de la synthèse de médiateurs lipidiques inflammatoires (Cox-2, Pges),
- d'augmenter l'expression d'un médiateur (Tgfb) et d'une enzyme de la synthèse de médiateurs lipidiques anti-inflammatoires (Alox15)
- d'augmenter l'expression d'enzymes des défenses antioxydantes (Sod2, Nqo1).

En conclusion, chez la souris âgée, une préparation aqueuse d'astragale caractérisée et titrée en polysaccharides impacte le système immunitaire à l'état basal : il réduit le statut inflammatoire, augmente les défenses antioxydantes et prépare à une défense anti-infectieuse. Cet effet pourrait contribuer à limiter les symptômes associés à l'inflammation latente caractéristique de l'âge et à améliorer l'efficacité de la réponse anti-infectieuse.

### Exemple 2 : activité de la préparation aqueuse d'astragale sur le système immunitaire d'animaux âgés lors d'une réponse anti-infectieuse

Des souris mâles C57BL/6 âgées de 18 mois ont reçu par gavage quotidien une dose de 40mg/kg de la préparation aqueuse d'astragale.

Au bout de 15 jours, les macrophages péritonéaux d'une partie des souris sont récupérés et exposés à des pathogènes (une bactérie, une levure) et à des substances antigéniques (un composé de paroi de levure [zymosan] et un analogue de matériel génétique viral [Poly (I :C)]).

Les capacités microbicides des macrophages vis-à-vis des pathogènes et des substances antigéniques sont évaluées (production d'espèces réactives de l'oxygène, impact sur la croissance des pathogène, capacité de phagocytose).

L'autre partie des souris est traitée par une injection unique intrapéritonéale de LPS (lipopolysaccharide bactérien) pour induire un péritonite. 12h après, le sang et le liquide péritonéal sont récupérés pour analyser plusieurs paramètres caractéristiques de l'état du système immunitaire : détermination par cytométrie de flux des populations immunitaires (nombre et proportion), dosage par ELISA de médiateurs de la réponse inflammatoire, expression de gènes impliqués dans l'inflammation et le stress oxydant par RT-QPCR.

Des groupes de souris contrôles *ad hoc* sont employés comme comparateur pour évaluer l'impact de la préparation d'astragale.

Sur des macrophages péritonéaux issues de souris traitées par la préparation aqueuse d'astragale, on constate :
- une augmentation de la production d'espèces réactives de l'oxygène lors d'un exposition à des pathogènes (*S*. *pneumoniae, C.albicans*) ou à une substance antigénique (composé de paroi de levure [zymosan]),
- une réduction de la prolifération bactérienne de *S. pneumoniae,*
- une augmentation de la capacité de phagocytose d'un analogue de matériel génétique viral [Poly (I:C)].

Chez des souris âgées avec une péritonite au LPS, la préparation aqueuse d'astragale a pour effet°:
- de moduler les populations immunitaires innées et adaptatives en faveur d'une réponse moins inflammatoire en lien avec de meilleures capacités d'élimination de pathogènes (augmentation des macrophages classiques, baisse des macrophages patrouilleurs et intermédiaire, des Treg, Th17, cellules dendritiques inflammatoires,
- d'induire une moindre augmentation du niveaux de médiateurs inflammatoires (IL-6, TNF-a)
- d'augmenter une médiateur anti-inflammatoire (IL-10).

En conclusion, chez la souris âgée, une préparation aqueuse d'astragale caractérisée et titrée en polysaccharides impacte le système immunitaire : les capacités d'une réponse anti-infectieuse semblent plus efficaces (meilleure capacité d'élimination de agents infectieux, environnement Th1 favorable au fonctionnement des macrophages) et la réponse inflammatoire est mieux équilibrée (modulation de l'inflammation et de la réponse oxydante). Cet effet pourrait contribuer à limiter la vulnérabilité aux infections et les complication associées.

Exemple 3 : activité d'une préparation aqueuse d'astragale dans la réponse immunitaire antigrippale *in vivo* chez l'animal âgé

### Matériel et méthodes

### Traitement des animaux

Des souris mâles C57BL/6 âgées de 20 mois ont reçu par gavage quotidien une dose de 40mg/kg d'une préparation aqueuse d'astragale. Au bout de 15 jours, les souris sont vaccinées contre la grippe saisonnière avec le vaccin monovalent 2009 H1N1 (pdm09). Pendant les 7 jours suivant la vaccination, les souris sont gavées quotidiennement avec la même dose d'extrait d'astragale.

Des groupes de souris contrôles *ad hoc* sont également inclus (souris âgées non gavées et vaccinées ; souris jeunes gavées ou non, et toutes vaccinées).

### Analyse sérologique

La séroconversion est évaluée dans tous les groupes à différents temps via des prélèvements sanguins réalisés : 1 jour avant la vaccination, à 5, 10, 30 et 50 jours après la vaccination.

A ces temps, les dosages sériques en IgG anti-hémagglutinine A sont réalisés par la méthode ELISA.

### Résultats

Si l'administration par voir orale de la préparation aqueuse d'astragale n'a pas d'effet chez les animaux jeunes sur la production d'IgG anti-hémagglutinine A en réponse à la vaccination au cours du temps, chez les souris âgées on constate en revanche une augmentation de la production d'anticorps à partir de J 30 (différence non significative avec le groupe de souris contrôles âgées vaccinées).

A J 50, cette augmentation de la production d'IgG est significativement supérieure à celle du groupe de souris contrôles âgées vaccinées, et est aussi élevée que dans le groupe des souris jeunes.

### Conclusion

Chez la souris âgée, la préparation aqueuse d'astragale caractérisée et titrée en polysaccharides impacte la réponse du système immunitaire lors d'une vaccination antigrippale : un potentiel effet adjuvant est montré via une augmentation la quantité d'IgG anti-hémagglutinine A produite.

L'âge avancé étant caractérisé par un défaut de réponse vaccinale, l'administration orale de cette préparation aqueuse caractérisée et titrée en polysaccharides, pourrait avoir un effet adjuvant à la vaccination en améliorant la production d'anticorps, et plus particulièrement la réponse IgG à la vaccination antigrippale chez ces sujets fragiles immunosénescents.

## Revendications

1. Préparation d'une plante du genre *Astragalus,* de préférence sous forme d'un préparation aqueuse, pour son utilisation comme immunorégulateur et/ou immunomodulateur chez des sujets humains ou animaux.

2. Préparation pour son utilisation, selon la revendication 1, dans laquelle ladite utilisation comme immunorégulateur et/ou immunomodulateur comprend une utilisation comme immunorégulateur et/ou immunomodulateur du système immunitaire en situation basale.

3. Préparation pour son utilisation, selon l'une quelconque des revendications 1 ou 2, dans laquelle ladite utilisation comme immunorégulateur et/ou immunomodulateur comprend une utilisation comme immunorégulateur et/ou immunomodulateur du système immunitaire lors d'une réponse immunitaire anti-infectieuse, en particulier bactérienne et/ou virale.

4. Préparation pour son utilisation, selon l'une quelconque des revendications 1 à 3, dans laquelle ladite utilisation comme immunorégulateur et/ou immunomodulateur comprend une utilisation comme immunorégulateur et/ou immunomodulateur du système immunitaire lors d'une vaccination.

5. Préparation pour son utilisation, selon l'une quelconque des revendications 1 à 4, dans laquelle les sujets sont choisis chez les sujets âgés, avantageusement ayant atteint les deux tiers de leur durée de vie.

6. Préparation pour son utilisation, selon la revendication 5, dans laquelle les sujets sont choisis parmi les sujets humains âgés, par exemple les sujets humains ayant au moins 50 ans, de préférence au moins 60 ans, de préférence au moins 65 ans.

7. Préparation pour son utilisation, selon l'une quelconque des revendications 1 à 6, dans laquelle ladite plante du genre *Astragalus* est choisie parmi *Astragalus alopecuroides L., Astragalus alopecurus Pall., Astragalus alpinus L., Astragalus australis (L.) Lam., Astragalus austriacus Jacq., Astragalus baionensis Loisel., Astragalus boeticus L., Astragalus cicer L., Astragalus crenatus Schult., Astragalus frigidus L., Astragalus glycyphyllos L., Astragalus hamosus L., Astragalus hypoglottis L., Astragalus membranaceus var mongholicus* (Bunge) P.K. Hsiao , *Astragalus membranaceus* (Fisch.) Bunge, *Astragalus membranaceus* Moench, *Astragalus monspessulanus L., Astragalus mongholicus* Bunge, *Astragalus tragacantha L., Astragalus onobrychis L., Astragalus sempervirens Lam.*

8. Préparation pour son utilisation, selon l'une quelconque des revendications 1 à 7, dans laquelle ladite préparation est issue d'une plante sèche ou fraîche.

9. Préparation pour son utilisation, selon l'une quelconque des revendications 1 à 8, dans laquelle ladite préparation est issue de la racine de ladite plante.

10. Préparation pour son utilisation, selon l'une quelconque des revendications 1 à 9, dans laquelle ladite préparation est issue d'un procédé comprenant les étapes suivantes :
- une étape de broyage de la plante, dont les particules présentent avantageusement des dimensions allant de 5 mm à 10 mm,
- au moins une étape d'extraction dans un milieu aqueux, pendant au moins 1h, par exemple allant de 1h à 10h, à une température allant de 50 à 90°C,
- une étape de filtration pour récupérer une préparation aqueuse intermédiaire,
- une étape de concentration (par exemple évaporateur rotatif) et de séchage (par exemple lyophilisation ou atomisation), pour obtenir ladite préparation aqueuse.

11. Composition pharmaceutique ou non-pharmaceutique, contenant une préparation selon l'une quelconque des revendications 1 à 10, pour son utilisation comme immunorégulateur et/ou immunomodulateur chez des sujets humains ou animaux.

12. Composition selon la revendication 11, contenant en outre l'un au moins des ingrédients suivants : au moins une préparation d'algue, au moins une préparation de plante, des probiotiques, des postbiotiques, des vitamines, des minéraux ou tout autre ingrédient nutritionnel.

13. Composition selon la revendication 12, adaptée à une utilisation non-thérapeutique dans l'industrie cosmétologique, agro-alimentaire, de la nutrition, des compléments alimentaires, de la nutrition animale.
